# EUROPEAN PATENT APPLICATION

(11) **EP 1 172 101 A1**
(43) Date of publication of application: **16.01.2002**
(21) Application number: 01114212.2
(22) Date of filing: 12.06.2001
(51) Int. Cl.: A61K 31/18, A61K 31/63, A61P 17/06

(54) **The use of nimesulide for the treatment of psoriasis and psoriatic arthritis**

(30) Priority: 20.06.2000 US 212675 P
(71) Applicant: Helsinn Healthcare S.A., 6912 Pazzallo-Lugano (CH)
(72) Inventor: Villa, Giuliana, 6912 Pazzallo-Lugano (CH); Macchi, Fabio, 6912 Pazzallo-Lugano (CH); Sarzi-Puttini, Piercarlo, 6912 Pazzallo-Lugano (CH)
(74) Representative: Minoja, Fabrizio, Dr.

(57) **Abstract**

The invention relates to the use of Nimesulide or of a physiologically equivalent form in the treatment of psoriasis or of psoriatic arthritis.

## Description

The present invention relates to the use of Nimesulide or of a physiologically equivalent form thereof in the treatment of psoriasis and of psoriatic arthritis.

The invention also relates to the use of Nimesulide or of a physiologically equivalent form thereof for the preparation of medicaments for the treatment of psoriasis or psoriatic arthritis.

Psoriatic arthritis is a rheumatoid-like arthritis, usually negative for the rheumatoid factor, associated to classic psoriasis of the skin or nails. This disease is present in up to 0.1% of world population and it usually begins between 30-50 years of age, in both sexes.

Psoriatic arthritis (PA) primarily involves the distal interphalangeal joints of fingers or toes. Asymmetric involvement of large and small joints, as well as of sacroiliac joints and spine, is common. PA can at times be quite destructive, progressing to chronic arthritis and arthritis mutilans, with extensive destruction of large and small joints. The clinical course of PA is long term, with characteristic remissions and relapses. In most cases, psoriasis may precede the onset of psoriatic arthritis, with a vary variable latency time (up to 10 years), however there are also forms in which psoriatic arthritis may precede the onset of psoriasis.

The exact cause of the disease is unknown, although interplay of immune, genetic and environmental factors are suspected.

Treatment is directed at control of skin lesions and joint inflammation. Pharmacological therapy is similar to that used for rheumatoid arthritis, and it is mainly based on the use of nonsteroidal anti-inflammatory drugs (NSAIDs) or steroids. Such drugs exert an effective action on flogosis and pain, but they should however be used with extreme caution since, in addition to their well-known side effects, they may cause skin lesions to exacerbate and become pustular.

The other drugs used in the treatment of PA induce adverse side effects as well:
Gold compounds are somewhat beneficial, but they may cause toxic effects and are contraindicated in patients with hepatic or renal disease;
Penicillamine exerts beneficial effects similar to those of gold compounds, but it may induce side effects requiring discontinuation, such as marrow suppression, nephrosis, proteinuria etc;
Sulfasalazine is quite effective, but it may cause neutropenia, hemolysis and hepatitis.

Cytotoxic or immunosuppressive drugs, such as Azathioprine and Methotrexate, may only be used in severe cases of the diseases, since they induce major side effects, such as bone marrow suppression, liver disease, pneumonitis.

Etretinate may be effective in severe psoriasis, but it can induce hypervitaminosis A, teratogenicity, hepatic toxicity.

It is therefore quite evident the need for a drug which is effective on both the skin disease and the inflammatory one, without inducing the severe side effects mentioned above.

Nimesulide is a NSAID that has been used for some time in the treatment of a variety of inflammatory and pain conditions. Nimesulide acts by a selective inhibition of prostaglandin biosynthesis.

It has now surprisingly been found that Nimesulide exerts a beneficial action on joint flogosis without negatively affecting the skin lesions as the other NSAIDs do, but on the contrary inducing a remarkable improvement of such lesions.

According to the invention, Nimesulide can be administered by the oral, topical, parenteral or rectal route, the oral and topical routes being particularly preferred, optionally in combination one with the other.

Nimesulide or a physiologically equivalent form thereof will be administered at dosages ranging from 100 to 400 mg, once or twice daily, by the oral, parenteral or rectal routes. When Nimesulide or its physiologically equivalent form is administered by the topical/transdermal route, an application of a suitable topical administration form containing from 1 to 20 % by weight of active ingredient will be applied on the affected skin once or twice a day.

The oral and topical routes are particularly preferred.

Examples of physiologically equivalent forms of Nimesulide include salts such as that disclosed in EP-A-937709, cyclodextrin complexes ( WO94/02177) or other forms, which are converted into Nimesulide or active metabolites thereof after administration.

Nimesulide or said equivalent forms will be conveniently administered in form of tablets, capsules, granulates, solutions or suspensions, suppositories, vials.

For the topical administration, suitable compositions include creams, gels, ointments, solutions, powders, patches and the like. Examples of preferred topical compositions are disclosed in WO 96/11002, EP-A-1007001, WO 98/37879, which are herein incorporated by reference.

Controlled double-blind clinical trials were carried out on patients 18 to 70 years old affected by psoriatic arthritis showing at least three swollen joints, absence of rheumatoid factor and no other rheumatic conditions.

The patients, who received no previous pharmacological treatment with antirheumatic drugs during the three-month pre-study period, were treated for 4 weeks with Nimesulide (100 to 400 mg/day orally or application of a 3% gel twice a day).

At the end of the treatment period, the following parameters were evaluated:
- number of tender and swollen joints
- pain score
- morning stiffness
- skin symptoms evaluated according to the Psoriasis Area Severity Index (PASI)
- subjective evaluation.

Nimesulide turned out to be clinically effective in the treatment of oligopolyarticular psoriatic arthritis in a statistically significant way. The treatment caused no toxic or untoward effect, particularly no gastro-intestinal adverse effect, in agreement with the data from studies showing good tolerability of Nimesulide.

## Claims

1. The use of Nimesulide or of a physiologically equivalent form thereof for the preparation of medicaments for the treatment of psoriasis and psoriatic arthritis.

2. The use as claimed in claim 1, wherein the physiologically equivalent form of Nimesulide is a salt or a cyclodextrin complex.

3. The use as claimed in claim 1 or 2 wherein Nimesulide or its physiologically equivalent form are administered orally.

4. The use as claimed in claim 1 or 3 wherein Nimesulide or its physiologically equivalent form are administered in form of tablets, granules, capsules, suspensions.

5. The use as claimed in claim 1 or 3 wherein Nimesulide or its physiologically equivalent form are administered at dosages ranging from 100 to 400 mg.

6. The use as claimed in claim 1 or 2 wherein Nimesulide or its physiologically equivalent form are administered topically.

7. The use as claimed in claim 5 wherein Nimesulide or its physiologically equivalent form are administered in form of creams, gels, ointments, solutions, powders, patches.
